# EUROPEAN PATENT SPECIFICATION

(11) **EP 4 081 271 B1**
(45) Date of publication and mention of the grant of the patent: **12.02.2025**
(21) Application number: 20839101.1
(22) Date of filing: 23.12.2020
(51) Int. Cl.: A61L 27/24, A61L 27/38, A61L 27/52

(54) **OPHTHALMOLOGICAL DEVICE FOR THE TREATMENT OF LSCD AND SUBSTRATE FOR USE IN SAME**
OPHTHALMOLOGISCHE VORRICHTUNG ZUR BEHANDLUNG VON LSCD UND SUBSTRAT ZUR VERWENDUNG DARIN
DISPOSITIF OPHTALMOLOGIQUE POUR LE TRAITEMENT DE LSCD ET SUBSTRAT DESTINÉ À ÊTRE UTILISÉ DANS CELUI-CI

(30) Priority: 24.12.2019 EP 19219707
(43) Date of publication of application: 02.11.2022
(73) Proprietor: Universiteit Antwerpen, 2000 Antwerpen (BE); Universitair Ziekenhuis Antwerpen, 2650 Edegem (BE)
(72) Inventor: TASSIGNON, Marie-José, 2600 Berchem (BE); HAAGDORENS, Michel, 2650 Edegem (BE)
(74) Representative: Beck, Michaël Andries T.
(86) International application number: PCT/EP2020/087811
(87) International publication number: WO 2021/130334

(56) References cited:
- US-A1- 2005 080 484
- MICHEL HAAGDORENS ET AL: "In Vitro Cultivation of Limbal Epithelial Stem Cells on Surface-Modified Crosslinked Collagen Scaffolds", STEM CELLS INTERNATIONAL, vol. 2019, 1 April 2019 (2019-04-01), US, pages 1 - 17, XP055703575, ISSN: 1687-966X, DOI: 10.1155/2019/7867613

## Description

### Background

The corneal limbus (hereinafter "limbus") is the border of the cornea and the sclera. The limbus contains niches that normally harbor a stem cell population. Limbal stem cell deficiency (LSCD) is an orphan disease that leads to corneal blindness. In LSCD, the epithelial stem cells that repopulate the corneal epithelium are lost, leading to corneal neovascularization, ocular inflammation, all of which result in corneal opacification and ocular pain. Current treatment of choice for LSCD is Cultivated Limbal Epithelial Transplantation (CLET), in which cultured epithelial cells are transplanted to the patient's diseased eye.

The stem cell carrier most frequently used in these trials is the Human Amniotic Membrane (HAM). However, the use of HAM in corneal tissue engineering is limited due to inherent shortcomings of this biological membrane. Hence, there is a need for alternatives to HAM for the cultivation of Limbal Epithelial Stem Cells (LESC).

International patent application publication no. WO 2015/032985 A1 in the name of UAB Ferentis discloses a hydrogel derived from a methacrylated or acrylated natural polymer and a synthetic polymer, and a method of preparing the same. The application further relates to 3D scaffolds and implants comprising said hydrogel.

International patent application publication no. WO 2015/055656 A1 in the name of UAB Ferentis discloses a corneal implant comprising a matrix material and a core part wherein the core part is arranged essentially in the middle of the matrix material. The core part is anti-fouling in order to stop cell proliferation across said part. It further relates to a method of preparing the said product and the use of the same.

International patent application publication no. WO 2015/055661 A1 in the name of UAB Ferentis discloses a method of surface modifying a hydrogel using microcontact printing or photolithography or inkjet and hydrogels obtained by said method.

The article by H.J. Levis and J.T. Daniels, "Recreating the Human Limbal Epithelial Stem Cell Niche with Bioengineered Limbal Crypts", Curr. Eye Res. 2016 Sep 4; 41(9):1153-60 discloses a process to produce collagens and to impart a pattern of grooves over the entire surface of the resulting collagen suspension.

The article by I. Ortega et al., "Combination of Microstereolithography and Electrospinning to Produce Membranes Equipped with Niches for Corneal Regeneration", J. Vis. Exp. (91), e51826, doi:10.3791/51826 (2014), discloses the experimental fabrication of PLGA biodegradable membranes equipped with micropockets based on a microstereolithographically produced PEGDA template, which are proposed for use as an artificial limbus.

United States patent application publication no. US 2005/080484 A1 discloses a corneal appliance that is placed over an eye, having a lens body and epithelial cells secured over the lens body. The epithelial cells of the appliance may be derived from cultured cells, including stem cells, such as limbal stem cells, or epithelial cell lines, or may include at least a portion of the epithelium of the eye on which the appliance is placed. The corneal appliance may have a cellular attachment element between the lens body and the epithelial cells to facilitate attachment of the epithelial cells over the lens body. The corneal appliance is intended to be used on a deepithelialized eye, which may be an eye that has had the epithelium fully or partially removed. The corneal appliance may be used to improve vision. Methods of producing the corneal appliance and of improving vision are also disclosed.

### Summary of the Invention

According to an aspect of the present invention, there is provided an ophthalmological device for the treatment of Limbal Stem Cell Deficiency, the device comprising:
- a stem cell carrier substrate; and
- a culture of limbal epithelial stem cells cultivated on said stem cell carrier substrate;
wherein said stem cell carrier substrate comprises a hydrogel containing collagen or collagen-mimicking peptides; and wherein a ring-shaped area on a surface of said stem cell carrier substrate is provided with a pattern of niches, positioned so as to coincide, after transplantation, with the limbus.

The term "hydrogel" is used herein to denote a gel of hydrophilic natural or synthetic polymers where the dispersion media is water.

The term "device" is used herein to denote the hydrogel-based stem cell carrier substrate, loaded with a culture of limbal epithelial stem cells, in particular for use as an ophthalmological implant. The stem cell carrier substrate is capable of retaining its shape in the absence of external forces. The material of the substrate must be sufficiently rigid to be able to retain the pattern of niches. However, in view of its being made of a hydrogel, the substrate is sufficiently flexible to adapt as much as necessary to the shape (in particular the surface curvature) of the eye to which it is applied.

It is known that collagen molecules can be covalently cross-linked, leading to the formation of a stable hydrogel. Chemically crosslinked collagen materials have been engineered for biomedical applications in this way, including to produce an artificial cornea. While collagen hydrogels may therefore be considered promising in terms of bio-compatibility with the cornea, they have not been successfully applied as implants for the treatment of LSCD in CLET.

It is an advantage of the collagen hydrogel based devices according to the present invention that they are less costly and easier to procure than the known alternative materials. This also applies to hydrogels containing collagen-mimicking peptides. Throughout the remainder of the description, the term "collagen" is to be understood as also referring to collagen-mimicking peptides, unless stated otherwise.

The collagen hydrogel based devices according to the present invention have been shown to exhibit better optical properties than control devices based on Human Amniotic Membrane (HAM) or Recombinant Human Collagen (RHC) Type I (isotropic or anisotropic). The collagen hydrogel based devices according to the present invention also outperform isotropic and anisotropic RHC-I devices on the point of degradation resistance.

Under physiological conditions, healthy limbal epithelial stem cells reside in stem cell niches (limbal crypts within the palisades of Vogt macrostructure). It has been found that when the known CLET techniques are applied, these stem cell niches are not regenerated in the patient after the transplantation. The present invention is based *inter alia* on the insights of the inventors that by recreating a similar pattern of niches in a collagen hydrogel CLET device, the niche structure can be preserved after the transplantation of the device, leading to improved overall success of the CLET intervention. Hence, a similar pattern of niches can simulate or mimic the stem cell niches in the palisades of Vogt macrostructure.

The patterned portion of the device is expected to increase cell yield after cultivation and increased long-term cell survival of transplanted stem cells. The use of a device with a pattern of niches is therapeutically promising for certain classes of patients which have a low probability of success with existing CLET techniques, such as patients suffering a loss of naturally occurring niches through congenital conditions or severe burns.

The presence of niches is believed to increase the cell cultivation yield because it leads to a larger substrate surface. The cells in cultivation first fill up the niches, before proliferating further over the flat surface thus formed.

In addition to allowing growth of stem cells on its patterned surface, the biomaterial substrate of the device according to embodiments of the present invention may be loaded with diverse pharmaceutical components that may be deemed therapeutically useful to optimize the outcome.

The pattern may in particular be a three-dimensional pattern, i.e. a pattern presenting depth variations, as seen in a direction normal to the surface of the device. The pattern may be a pattern of grooves and ridges. Alternatively, the pattern may be a pattern of indentations, e.g. pyramidal indentations or hexagonal indentation (such as a honeycomb pattern). Preferably, the pattern mimics the complex three-dimensional structure of the native limbus, by presenting a macrostructure of grooves and ridges, and a microstructure of additional indentations, grooves, pockets and/or bulbs.

The pattern may be provided on the side of the device that faces the air/cornea after implantation. The pattern may also be provided on the side that faces the ocular tissue after implantation. Optionally, patterns may be provided on both sides.

The present invention is further based on the insight of the inventors that by providing the pattern of niches only over a ring-shaped area of the surface of the device, a niche-free central zone can be retained in the corneal area, thus avoiding any adverse effects on the image forming function of the eye. The inventors have found that they could obtain this specific structure by using a collagen-based hydrogel, which is sufficiently malleable to permit applying a very fine and accurately localized pattern to it by pressing it onto a mold.

The present invention is further based on the insight that the pattern of niches has to coincide with the limbus, after transplantation.

In embodiments of the ophthalmological device according to the present invention, said ophthalmological device, or the stem cell carrier substrate of the device, is configured to be implanted at the level of the limbus. More in particular, the periphery or a rim portion of the stem cell carrier substrate is configured to be implanted at the level of the limbus. Said periphery or rim portion is thereby configured to cover, contact and/or to be attached to the limbus.

Advantageously, providing the periphery of the substrate with the pattern of niches allows replenishing the limbus with stem cells, allows controlling proliferation of fibro-vascular tissue and helps avoiding corneal conjunctivalisation. Consequently, sustainable cellularization of limbal stem cells can be obtained, which offers a long-term solution for severe cases of limbal stem cell niche deficiencies.

In alternative embodiments of the ophthalmological device according to the present invention, said ophthalmological device, or the stem cell carrier substrate of the device, is configured to be implanted at the level of the sclera. More in particular, the periphery or a rim portion of the stem cell carrier substrate of the device is configured to be implanted at the level of the sclera. Said periphery or rim portion is thereby configured to cover, contact and/or to be attached to the sclera. According to these embodiments, the ophthalmological device or stem cell carrier substrate are preferably implanted in a scleral pocket.

In embodiments of the ophthalmological device according to the present invention, said ophthalmological device is implanted on the de-epithelialized eye. The ophthalmological device may be implanted after removal of pannus and/or partial stromal lamellar keratectomy.

In an embodiment of the ophthalmological device according to the present invention, the stem cell carrier substrate has a shape of a dome, wherein a rim portion of said dome presents said niches and wherein a central portion of said dome is devoid of said niches. The rim portion thus represents the ring-shaped area described above.

In this embodiment, the dome presents a smooth central window, free of patterning, surrounded by a patterned rim. The production of the device in a round dome-shape helps in better mimicking the native anterior corneal surface.

The central window portion of the device may comprise a different material, e.g. a different cross-linked collagen hydrogel, than the rim portion.

In an embodiment of the ophthalmological device according to the present invention, the stem cell carrier substrate has a shape of a ring. The entire ring itself, or a portion thereof, represents the ring-shaped area described above.

Compared to the previously described "dome" embodiment, this embodiment substantially only includes the rim portion. It is an advantage of this embodiment that the cornea is not covered by the device after transplantation. The ring is preferably shaped so as to cover the limbus region of the eye. Accordingly, for use in human patients, the ring preferably has a diameter in the range of 10-14 mm, more preferably in the range of 11-12 mm. The ring preferably follows the curvature of the surface of the eye.

In embodiments of the ophthalmological device according to the present invention, the periphery or rim portion of the stem cell carrier substrate of the device comprises biocompatible materials and may be nontransparent, e.g. glazy, tinted or opaque. In particular embodiments, the periphery or rim portion may be composed of a composition comprising components for at least partially blocking light.

Due to its position on the eye, the periphery or a rim portion cannot affect light transmission into the eye. Hence, by using nontransparent materials for the periphery of the substrate, the cells cultured by the limbus may advantageously be protected from being harmed by light.

In an embodiment of the ophthalmological device according to the present invention, said stem cell carrier substrate comprises a 12% Collagen-Like Peptide, CLP-12, hydrogel.

In a particular embodiment, said CLP-12 hydrogel is crosslinked with 1-ethyl-3-(3-dimethyl aminopropyl) carbodiimide, EDC.

In another particular embodiment, said CLP-12 hydrogel is crosslinked with 4-(4,6-dimethoxy-1,3,5-triazin-2-yl)-4-methyl-morpholinium chloride, DMTMM.

This particular hydrogel presents very good primary LESC cultivation and very good cell proliferation.

In an embodiment of the ophthalmological device according to the present invention, said stem cell carrier substrate comprises a 18% Collagen-Like Peptide, CLP-18, hydrogel, cross-linked with 4-(4,6-dimethoxy-1,3,5-triazin-2-yl)-4-methyl-morpholinium chloride, DMTMM.

This particular hydrogel presents very good primary LESC cultivation and very good cell proliferation.

In an embodiment of the ophthalmological device according to the present invention, said stem cell carrier substrate comprises an RHC Type I based hydrogel.

RHC I may be derived from plants and presents a promising biomaterial for ocular tissue engineering. It can successfully be processed into hydrogels of which collagen orientation is influenced by application of shear force. RHC I implants have proved to be suitable substrates for further functionalization via surface patterning of other proteins (fibronectin), enabling advanced implant development. RHC I hydrogels are superior in terms of transparency, mechanical handling, standardization and microbial resistance when compared to HAM. RHC I hydrogels are found to be safe and biocompatible *in vitro* and *in vivo.*

In embodiments of the ophthalmological device according to the present invention, the periphery or rim portion of the stem cell carrier substrate of the device may be at least partially undulated or corrugated.

In an embodiment of the ophthalmological device according to the present invention, said pattern consists of regularly spaced grooves.

The grooves of this embodiment accurately and efficiently mimic the naturally occurring niches in which the healthy limbal epithelial stem cells reside in natural physiological conditions.

In a particular embodiment, said regularly spaced grooves have a width in the range of 15-150 um, preferably in the range of 50-90 µm, more preferably in the range of 60-80 µm, most preferably in the range of 65-75 um. The width may in particular be 70 um or thereabout.

The inventors have found through Fourier Domain-OCT imaging of healthy subjects that the limbal niches in the superior and inferior limbal region have a mean groove width of 72 µm for the palisades of Vogt, with significant variation attributable to iris color and age. It is therefore believed to be advantageous to adapt the attributes of the artificial niches of the device according to the present invention to those of the naturally occurring niches.

In an embodiment of the ophthalmological device according to the present invention, said substrate is loaded with one or more pharmaceutical components.

It is an advantage of this embodiment that the implant may be used to bring therapeutically beneficial pharmaceutical components directly to the implant site. These components may include, without limitation, one or more of cytokines, growth factors, enzymes, and proteins. Components may *inter alia* be selected to obtain immunosuppressive effects, anti-inflammatory effects, cell growth stimulation, anesthetic effects, etc.

According to an aspect of the present invention, there is provided a stem cell carrier substrate for use in the ophthalmological device as described above, the stem cell carrier substrate comprising a hydrogel containing collagen or collagen-mimicking peptides.

According to an aspect of the present invention, there is provided a method for producing the ophthalmological device as described above, the method comprising:
- chemically crosslinking collagen to produce said hydrogel forming said stem cell carrier substrate;
- pressing a stamp onto said hydrogel, said stamp having a template of said ring-shaped area provided with said pattern of niches; and
- applying limbal epithelial stem cells to said molded hydrogel.

According to an aspect of the present invention, there is provided a use of the ophthalmological device as described above as an implant for the treatment of Limbal Stem Cell Deficiency. The ophthalmological device may be so used in humans or in animals other than humans.

### Brief Description of the Figures

These and other features and advantages of embodiments of the present invention will now be describe in more detail with reference to the accompanying drawings, in which:
- Figure 1 is a schematic representation of a first embodiment of the device according to the present invention;
- Figure 2 is a schematic representation of a second embodiment of the device according to the present invention; and
- Figure 3 presents a flow chart of an embodiment of the process according to the present invention.

### Detailed Description of Embodiments

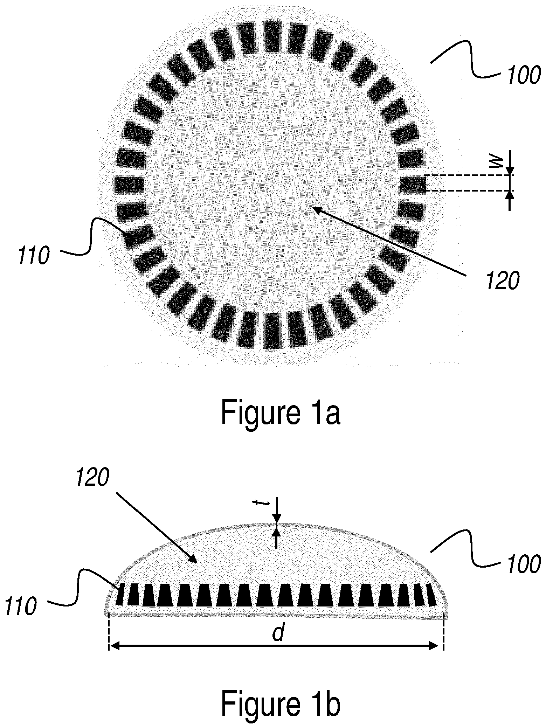

With reference to Figure 1, a device **100** according to a first embodiment of the present invention has a dome-shape, with a curvature resembling the native cornea. Figure 1a shows a schematic top view of the device. Figure 1b shows a schematic elevation of the device. The width of the niches or grooves **110** has been exaggerated for illustrative purposes - consequently, the number and spacing of the illustrated niches or grooves **110** is not meant to represent realistic values. The curvature of the dome has also been exaggerated for illustrative purposes.

The diameter d of the disk is preferably in the range of 10-14 mm, more preferably in the range of 11-12 mm. The hydrogel must have sufficient mechanical strength and thickness for stem cell transfer. The thickness t of the hydrogel can be adjusted according to the patient's need, normally ranging from 50 to 500 um. The outer skirt or rim of the device has a 3D groove pattern with a width *w* of the grooves **110** and ridges ranging from 50 to 90 µm, preferably around 70 µm. In the illustrated case, the grooves are located on the superficial side of the hydrogel (the convex side). Alternatively or additionally, niches can be located at the concave side of the outer skirt, facing the ocular tissue that has been freed of the fibro-vascular tissue, depending on the degree of primary destruction or absence of the limbal niches.

The niches or grooves **110** are positioned in a ring-shaped area at the periphery of the disk so as to coincide, after transplantation, with the limbus. A central portion **120** of the device **100** is free from niches and forms a clear window. After transplantation, this central window portion **120** covers the cornea and therefore any interference with the optical function of the eye should be avoided or at least minimized. Optionally, this central window portion **120** is made of a different material than the ring-shaped area bearing the grooves **110.** Preferably, the central window portion **120** is made of a biodegradable or resorbable material, which presents the advantage that the area over the cornea opens up to expose the cornea to air after the time required for the degradation or resorption of the material.

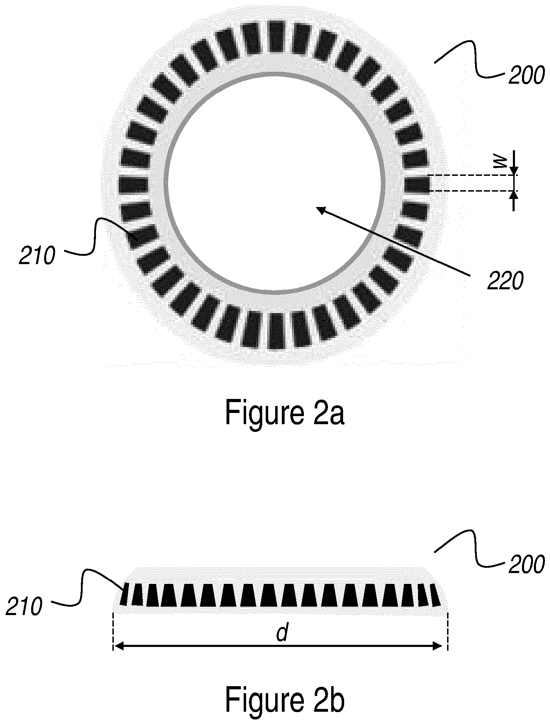

With reference to Figure 2, a device **200** according to a second embodiment of the present invention has a ring-shape, with a curvature resembling the native cornea. Figure 2a shows a schematic top view of the device. Figure 2b shows a schematic elevation of the device. The width of the niches or grooves **220** has been exaggerated for illustrative purposes - consequently, the number and spacing of the illustrated niches or grooves **200** is not meant to represent realistic values. The curvature of the ring has also been exaggerated for illustrative purposes.

The outer diameter d of the ring is preferably in the range of 10-14 mm, more preferably in the range of 11-12 mm. The outer diameter d of the ring may have any other value, based on measurements of the implantation site in the target patient. Other preferred parameters of the device are as discussed above in the context of Figure 1. The niches or grooves **210** are positioned on the ring-shaped device **200** so as to coincide, after transplantation, with the limbus. As the device **200** is ring-shaped, the central portion **220** is a void. After transplantation, this central portion **220** leaves the cornea uncovered.

Several collagen hydrogels may be used in the present invention. These include a 12% Collagen-Like Peptide (CLP-12) hydrogel, which may be crosslinked with, for example, 1-ethyl-3-(3-dimethyl aminopropyl) carbodiimide (EDC) or 4-(4,6-dimethoxy-1,3,5-triazin-2-yl)-4-methyl-morpholinium chloride (DMTMM); a 18% Collagen-Like Peptide (CLP-18) hydrogel, which may be crosslinked with, for example, DMTMM.

It is also possible to use hydrogels based on plant-based collagens, such as RHC Type I. The inventors have found that anisotropic RHC I hydrogels are less favorable for CLET than isotropic hydrogels (see also Figure 2, below).

The choice of the type of collagen to be used is constrained by the requirement that the resulting hydrogel be sufficiently solid to allow patterning. The inventors have found that patterning is generally successful in yeast-derived and synthetic collagens, but less so in collagens derived from Nicotiana tabacum plants.

While various specific collagen hydrogels have been discussed hereinabove, the skilled person will appreciate that the invention can also be practiced with various other chemical formulations of collagen (and peptide) hydrogels.

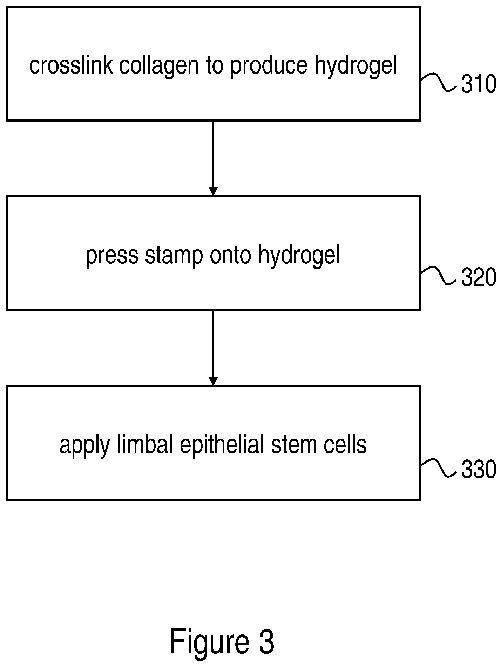

With reference to Figure 3, the invention also pertains to a method for producing the ophthalmological device as described above.

In a first step **310,** collagen is chemically crosslinked to produce the hydrogel forming the stem cell carrier substrate. Chemical crosslinking of collagen to produce a hydrogel is known in the art. For example, international patent application publication no. WO 2015/032985 A1 discloses a method of preparing a hydrogel comprising:
- providing a solution of a first polymer comprising a natural polymer comprising methacrylate and/or acrylate functional groups;
- providing a second polymer comprising a synthetic and/or a natural polymer having at least two functional groups selected from thiol, acrylate and/or methacrylate, or synthetic and/or natural monomers having thiol acrylate and/or methacrylate functional groups;
- mixing the first and the second polymer, or monomers, in water to a total polymer concentration of at least 2 weight%; and
- letting the functional groups of the first and the second polymer chains cross-link, optionally applying UV radiation to the mixture when the second polymer has acrylate and/or methacrylate functional groups.

The reader will find further information about this method in the cited publication. While this method of preparing the hydrogel is a preferred embodiment of the present invention, the invention is not limited thereto.

The hydrogel is formed into the desired dome shape or ring shape during or after the crosslinking step.

In a second step **320,** the pattern is applied by microcontact printing. A stamp presenting a template of the ring-shaped area provided with the desired pattern of niches is pressed onto the hydrogel (or vice versa). The stamp may be made of polydimethylsiloxane (PDMS) or any other suitable material. Preferably, the stamp is incubated, prior to contact with the hydrogel, with a suitable "ink" such as a 0.1 mg/mL human fibronectin dilution in phosphate-buffered saline.

In a third step **330,** the substrate is primed for cell cultivation by applying the initial (previously harvested or cultured) limbal epithelial stem cells to the molded (patterned) hydrogel. The device can be loaded with cultivated stem cells derived from the contralateral eye of the patient or from a highly compatible donor eye or other cells derived from stem cells. It shall be noted that the implant may be reloaded at a later stage (not illustrated) in a secondary procedure with cell cultures in case of early or late failure of the primary transplantation due to stem cell death.

The inventors' experiments have shown that surface patterning does not impact cell phenotype or genotype.

The present invention also pertains to the use of the ophthalmological device as described above as an implant for the treatment of Limbal Stem Cell Deficiency. The ophthalmological device may be so used in humans or in animals other than humans.

The present invention also pertains to a stem cell carrier substrate for use in the ophthalmological device as described above, the stem cell carrier substrate comprising a hydrogel containing collagen or collagen-mimicking peptides. A ring-shaped area on a surface of the stem cell carrier substrate is provided with a pattern of niches. All other optional features and technical effects disclosed above for the substrates that form part of embodiments of the ophthalmological device according to the invention, apply equally to the substrate as a separate inventive aspect. The substrate may produced according to steps **310** and **320** as described above in the context of Figure 3. Such a substrate can be produced and stored for a period of time before being used. Prior to implantation, it is primed for cell cultivation by applying the initial stem cells to the patterned area, as described in step **330** of Figure 3 above. The substrate may additionally be loaded with diverse pharmaceutical components that may be deemed therapeutically useful.

In a more general way, the present invention also pertains to a pre-formed substrate comprising a hydrogel containing collagen or collagen-mimicking peptides, the substrate being provided with a pattern of niches. Such a patterned substrate, when loaded with the appropriate payload (in particular, stem cells and/or pharmaceutical components) can be used as an implant in various other medical applications.

While the invention has been described hereinabove with reference to specific embodiments, this has been done to clarify and not to limit the invention, the scope of which is determined by the accompanying claims.

## Claims

1. An ophthalmological device (100, 200) for the treatment of Limbal Stem Cell Deficiency, the device (100, 200) comprising:
- a stem cell carrier substrate; and
- a culture of limbal epithelial stem cells cultivated on said stem cell carrier substrate;
wherein said stem cell carrier substrate comprises a hydrogel containing collagen or collagen-mimicking peptides; and
wherein a ring-shaped area on a surface of said stem cell carrier substrate is provided with a pattern of niches (110, 210), positioned so as to coincide, after transplantation, with the limbus.

2. The ophthalmological device (100) according to claim 1, wherein said stem cell carrier substrate has a shape of a dome, wherein a rim portion of said dome presents said niches and wherein a central portion (120) of said dome is devoid of said niches.

3. The ophthalmological device (200) according to claim 1, wherein said stem cell carrier substrate has a shape of a ring.

4. The ophthalmological device (100, 200) according to any of claims 1-3, wherein said stem cell carrier substrate comprises a 12% Collagen-Like Peptide, CLP-12, hydrogel.

5. The ophthalmological device (100, 200) according to claim 4, wherein said CLP-12 hydrogel is crosslinked with 1-ethyl-3-(3-dimethyl aminopropyl) carbodiimide, EDC.

6. The ophthalmological device (100, 200) according to claim 4, wherein said CLP-12 hydrogel is crosslinked with 4-(4,6-dimethoxy-1,3,5-triazin-2-yl)-4-methyl-morpholinium chloride, DMTMM.

7. The ophthalmological device (100, 200) according to any of claims 1-3, wherein said stem cell carrier substrate comprises a 18% Collagen-Like Peptide, CLP-18, hydrogel, cross-linked with 4-(4,6-dimethoxy-1,3,5-triazin-2-yl)-4-methyl-morpholinium chloride, DMTMM.

8. The ophthalmological device (100, 200) according to any of the claims 1-3, wherein said stem cell carrier substrate comprises a RHC Type I based hydrogel.

9. The ophthalmological device (100, 200) according to any of the preceding claims, wherein said pattern consists of regularly spaced grooves (120, 220).

10. The ophthalmological device (100, 200) according to claim 9, wherein said regularly spaced grooves (120, 220) have a width in the range of 15-150 µm, preferably in the range of 50-90 µm, more preferably in the range of 60-80 um, most preferably in the range of 65-75 µm.

11. The ophthalmological device (100, 200) according to any of the preceding claims, wherein said substrate is loaded with one or more pharmaceutical components.

12. A stem cell carrier substrate for use in the ophthalmological device according to any of the preceding claims, the stem cell carrier substrate comprising a hydrogel containing collagen or collagen-mimicking peptides;
wherein a ring-shaped area on a surface of said stem cell carrier substrate is provided with a pattern of niches (110, 210), positioned so as to coincide, after transplantation, with the limbus.

13. A method for producing the ophthalmological device according to any of claims 1-11, the method comprising:
- chemically crosslinking (310) collagen or collagen-mimicking peptides to produce said hydrogel forming said stem cell carrier substrate;
- pressing (320) a stamp onto said hydrogel, said stamp having a template of said ring-shaped area provided with said pattern of niches; and
- applying (330) limbal epithelial stem cells to said molded hydrogel.

14. The ophthalmological device according to any of claims 1-11 for use as implant in the treatment of Limbal Stem Cell Deficiency.

## Patentansprüche

1. Ophthalmologische Vorrichtung (100, 200) zur Behandlung von Limbusstammzellinsuffizienz, wobei die Vorrichtung (100, 200) Folgendes umfasst:
- ein Stammzellträgersubstrat; und
- eine Kultur von Limbus-Epithelstammzellen, die auf dem Stammzellträgersubstrat kultiviert werden;
wobei das Stammzellträgersubstrat ein Hydrogel umfasst, das Kollagen oder Kollagen-imitierende Peptide enthält; und
wobei ein ringförmiger Bereich auf einer Oberfläche des Stammzellträgersubstrats mit einem Muster von Nischen (110, 210) versehen ist, die so angeordnet sind, dass sie nach der Transplantation mit dem Limbus übereinstimmen.

2. Ophthalmologische Vorrichtung (100) nach Anspruch 1, wobei das Stammzellträgersubstrat die Form einer Kuppel aufweist, wobei ein Randabschnitt dieser Kuppel die Nischen aufweist und wobei ein zentraler Abschnitt (120) der Kuppel frei von den Nischen ist.

3. Ophthalmologische Vorrichtung (200) nach Anspruch 1, wobei das Stammzellträgersubstrat die Form eines Rings aufweist.

4. Ophthalmologische Vorrichtung (100, 200) nach einem der Ansprüche 1 bis 3, wobei das Stammzellträgersubstrat ein Hydrogel mit 12 % Kollagen-ähnlichem Peptid, CLP-12, umfasst.

5. Ophthalmologische Vorrichtung (100, 200) nach Anspruch 4, wobei das CLP-12-Hydrogel mit 1-Ethyl-3-(3-dimethylaminopropyl)carbodiimid, EDC, vernetzt ist.

6. Ophthalmologische Vorrichtung (100, 200) nach Anspruch 4, wobei das CLP-12-Hydrogel mit 4-(4,6-Dimethoxy-1,3,5-triazin-2-yl)-4-methyl-morpholiniumchlorid, DMTMM, vernetzt ist.

7. Ophthalmologische Vorrichtung (100, 200) nach einem der Ansprüche 1 bis 3, wobei das Stammzellträgersubstrat ein Hydrogel mit 18 % Kollagen-ähnlichem Peptid, CLP-18, vernetzt mit 4-(4,6-Dimethoxy-1,3,5-triazin-2-yl)-4-methyl-morpholiniumchlorid, DMTMM, umfasst.

8. Ophthalmologische Vorrichtung (100, 200) nach einem der Ansprüche 1 bis 3, wobei das Stammzellträgersubstrat ein auf RHC Typ I basierendes Hydrogel umfasst.

9. Ophthalmologische Vorrichtung (100, 200) nach einem der vorhergehenden Ansprüche, wobei das Muster aus regelmäßig beabstandeten Rillen (120, 220) besteht.

10. Ophthalmologische Vorrichtung (100, 200) nach Anspruch 9, wobei, die regelmäßig beabstandeten Rillen (120, 220) eine Breite im Bereich von 15-150 µm, vorzugsweise im Bereich von 50-90 um, besonders bevorzugt im Bereich von 60-80 µm, am meisten bevorzugt im Bereich von 65-75 µm aufweisen.

11. Ophthalmologische Vorrichtung (100, 200) nach einem der vorhergehenden Ansprüche, wobei das Substrat mit einer oder mehreren pharmazeutischen Komponenten beladen ist.

12. Stammzellträgersubstrat zur Verwendung in der ophthalmologischen Vorrichtung nach einem der vorhergehenden Ansprüche, wobei das Stammzellträgersubstrat ein Hydrogel umfasst, das Kollagen oder Kollagen-imitierende Peptide enthält; und
wobei ein ringförmiger Bereich auf einer Oberfläche des Stammzellträgersubstrats mit einem Muster von Nischen (110, 210) versehen ist, die so angeordnet sind, dass sie nach der Transplantation mit dem Limbus übereinstimmen.

13. Verfahren zur Herstellung der ophthalmologischen Vorrichtung nach einem der Ansprüche 1 bis 11, wobei das Verfahren Folgendes umfasst:
- chemisches Vernetzen (310) von Kollagen oder Kollagen-imitierenden Peptiden zur Herstellung des Hydrogels, das das Stammzellträgersubstrat bildet;
- Pressen (320) eines Stempels auf das Hydrogel, wobei der Stempel eine Schablone des ringförmigen Bereichs aufweist, der mit dem Nischenmuster versehen ist; und
- Aufbringen (330) von Limbus-Epithelstammzellen auf das geformte Hydrogel.

14. Ophthalmologische Vorrichtung nach einem der Ansprüche 1 bis 11 zur Verwendung als Implantat bei der Behandlung von Limbusstammzellinsuffizienz.

## Revendications

1. Dispositif ophtalmologique (100, 200) pour le traitement du Déficit en Cellules Souches Limbiques, le dispositif (100, 200) comprenant :
- un substrat porteur de cellules souches ; et
- une culture de cellules souches épithéliales limbiques cultivées sur ledit substrat porteur de cellules souches ;
dans lequel ledit substrat porteur de cellules souches comprend un hydrogel contenant du collagène ou des peptides mimant le collagène ; et
dans lequel une zone en forme d'anneau sur une surface dudit substrat porteur de cellules souches est pourvue d'un motif de niches (110, 210), positionné de manière à coïncider, après transplantation, avec le limbe.

2. Dispositif ophtalmologique (100) selon la revendication 1, dans lequel ledit substrat porteur de cellules souches a une forme de dôme, dans lequel une portion formant rebord dudit dôme présente lesdites niches et dans lequel une portion centrale (120) dudit dôme est dépourvue desdites niches.

3. Dispositif ophtalmologique (200) selon la revendication 1, dans lequel ledit substrat porteur de cellules souches a une forme d'anneau.

4. Dispositif ophtalmologique (100, 200) selon l'une quelconque des revendications 1 à 3, dans lequel ledit substrat porteur de cellules souches comprend un hydrogel de Peptides semblables à du Collagène à 12 %, CLP-12.

5. Dispositif ophtalmologique (100, 200) selon la revendication 4, dans lequel ledit hydrogel CLP-12 est réticulé avec du 1-éthyl-3-(3-diméthylaminopropyl) carbodiimide, EDC.

6. Dispositif ophtalmologique (100, 200) selon la revendication 4, dans lequel ledit hydrogel CLP-12 est réticulé avec du chlorure de 4-(4,6-diméthoxy-1,3,5-triazin-2-yl)-4-méthyl-morpholinium, DMTMM.

7. Dispositif ophtalmologique (100, 200) selon l'une quelconque des revendications 1 à 3, dans lequel ledit substrat porteur de cellules souches comprend un hydrogel de Peptides semblables à du Collagène à 18 %, CLP-18, réticulé avec du chlorure de 4-(4,6-diméthoxy-1,3,5-triazin-2-yl)-4-méthyl-morpholinium, DMTMM.

8. Dispositif ophtalmologique (100, 200) selon l'une quelconque des revendications 1 à 3, dans lequel ledit substrat porteur de cellules souches comprend un hydrogel à base de RHC de type I.

9. Dispositif ophtalmologique (100, 200) selon l'une quelconque des revendications précédentes, dans lequel ledit motif consiste en rainures régulièrement espacées (120, 220).

10. Dispositif ophtalmologique (100, 200) selon la revendication 9, dans lequel lesdites rainures régulièrement espacées (120, 220) ont une largeur dans la plage de 15 à 150 µm, de préférence dans la plage de 50 à 90 µm, plus préférablement dans la plage de 60 à 80 µm, et de manière très préférable dans la plage de 65 à 75 µm.

11. Dispositif ophtalmologique (100, 200) selon l'une quelconque des revendications précédentes, dans lequel ledit substrat est chargé avec un ou plusieurs composants pharmaceutiques.

12. Substrat porteur de cellules souches destiné à être utilisé dans le dispositif ophtalmologique selon l'une quelconque des revendications précédentes, le substrat porteur de cellules souches comprenant un hydrogel contenant du collagène ou des peptides mimant le collagène ;
dans lequel une zone en forme d'anneau sur une surface dudit substrat porteur de cellules souches est pourvue d'un motif de niches (110, 210), positionné de manière à coïncider, après transplantation, avec le limbe.

13. Procédé de production du dispositif ophtalmologique selon l'une quelconque des revendications 1 à 11, le procédé comprenant :
- la réticulation chimique (310) du collagène ou des peptides mimant le collagène pour produire ledit hydrogel formant ledit substrat porteur de cellules souches ;
- l'application par pressage (320) d'un tampon sur ledit hydrogel, ledit tampon ayant un modèle de ladite zone en forme d'anneau pourvue dudit motif de niches ; et
- l'application (330) de cellules souches épithéliales limbiques sur ledit hydrogel moulé.

14. Dispositif ophtalmologique selon l'une quelconque des revendications 1 à 11 destiné à être utilisé en tant qu'implant dans le traitement du Déficit en Cellules Souches Limbiques.
